# EUROPÄISCHE PATENTSCHRIFT

(11) **EP 1 985 264 B1**
(45) Veröffentlichungstag und Bekanntmachung des Hinweises auf die Patenterteilung: **18.05.2011**
(21) Anmeldenummer: 08007349.7
(22) Anmeldetag: 15.04.2008
(51) Int. Cl.: A61F 5/02

(54) **Orthese**
Orthosis
Orthèse

(30) Priorität: 23.04.2007 DE 102007019526
(43) Veröffentlichungstag der Anmeldung: 29.10.2008
(73) Patentinhaber: Otto Bock HealthCare GmbH, 37115 Duderstadt (DE)
(72) Erfinder: Wagner, Helmut, 37115 Duderstadt (DE); Lidolt, Klaus, 37115 Duderstadt (DE); Vollbrecht, Matthias, 37412 Herzberg (DE)
(74) Vertreter: Lins, Edgar

(56) Entgegenhaltungen:
- WO-A-2007/003148
- DE-U1-202004 015 328
- US-A- 4 245 627
- US-A- 4 976 257

## Beschreibung

Die Erfindung betrifft eine Orthese mit einer zur Anlage am Körper eines Orthesenträgers ausgebildeten formstabilen Basisplatte.

Derartige Orthesen sind in zahlreichen Ausführungsformen bekannt. Als Basisplatte kann dabei jede Platte dienen, die durch geeignete Befestigungsmittel flächig am Körper des Orthesenträgers anliegt. In einer Ausführungsform befindet sich eine ventrale Basisplatte im Taillenbereich, wobei ein Befestigungsgurt den Körper in diesem Taillenbereich umspannt. Dabei kann dorsal eine Rückenpelotte vorgesehen sein, die von dem Befestigungsgurt übergriffen wird oder die Befestigungseinrichtungen für die entsprechenden Enden des Befestigungsgurts aufweist, sodass der Befestigungsgurt den Körper zusammen mit der Rückenpelotte umspannt.

Ein Hauptanwendungsgebiet einer derartigen Orthese ist eine Hyperextensionsorthese, bei der von der Basisplatte in Höhenrichtung, also nach oben und/oder nach unten wenigstens eine weitere Stützpelotte weg ragt, die stabil mit der Basisplatte verbunden ist. Für eine komplette Hyperextensionsorthese wirkt mit der Rückenpelotte ventral eine obere Sternalpelotte und eine untere Symphysenpelotte zur Bildung einer Dreipunkt-Abstützung zusammen. Die Basisplatte hat dabei im Wesentlichen die Funktion, als Befestigungselement für den Befestigungsgurt und die beiden ventralen Pelotten zu fungieren. Eine derartige Hyperextensionsorthese ist beispielsweise durch DE 39 28 628 C1 bekannt. Derartige Orthesen werden in mehreren Größen geliefert, um durch unterschiedlich große Basisplatten eine Anpassung an den Körperumfang und einen bequemen Sitz der Orthese zu ermöglichen. Bei einer zu schmalen Basisplatte würden die Befestigungsgurte zu stark in das Gewebe des Patienten einschneiden, während eine zu breite Basisplatte zu einem unsicheren Sitz führen würde. Bei den bekannten Orthesen besteht die Basisplatte, ebenso wie die Pelotten, aus einem Metallteil, das mit einer Polsterauflage versehen ist. Für die Ausbildung der verschiedenen Größen der Orthese müssen daher unterschiedlich große Basisplatten vorrätig gehalten werden.

Durch US 4,976,257 ist eine Hyperextensionsorthese bekannt, bei der eine Basisplatte über vertikale Arme zu einer Sternalpelotte und einer Lymphysenpelotte ausgebildet ist. Die Basisplatte besteht dabei aus einem flächigen harten äußeren Kunststoff, der zum Körper hin mit einer Polsterschicht verbunden ist. Im Bereich der Sternalpelotte kann die Basisplatte mit einer Befestigungsöffnung versehen sein, über die eine separate Polsterplatte als Sternalpelotte befestigbar ist. Die Polsterplatte ist dabei nicht mit der Basisplatte fluchtend angeordnet, sondern ist zum Körper hin versetzt gegenüber der Basisplatte befestigt. Das Polsterelement dient als einzige Anlagefläche der Sternalpelotte.

Der vorliegenden Erfindung liegt die Aufgabe zugrunde, eine Orthese so auszubilden, dass sie eine verbesserte Handhabung und Lagerhaltung ermöglicht.

Diese Aufgabe wird erfindungsgemäß mit einer Orthese der eingangs erwähnten Art dadurch gelöst, dass die Basisplatte an wenigstens einer Seite einen Ansatz mit einer Befestigungseinrichtung aufweist, mit der an den Ansatz ein die Basisplatte vergrößerndes Verlängerungsstück fluchtend mit dem Ansatz der Basisplatte zur steifen Verbindung der Basisplatte mit dem Verlängerungsstück angesetzt ist.

Bei der erfindungsgemäßen Orthese ist somit die Basisplatte durch Ansetzen von Verlängerungsstücken vergrößerbar, sodass in Abhängigkeit davon, ob an die Basisplatte kein Verlängerungsstück oder ein, zwei oder drei Verlängerungsstücke usw. angebracht sind, die Anpassung der Basisplatte an die Körperform oder an die jeweils benötigte Funktion der Orthese realisierbar sind, ohne dass für jede realisierte Größe unterschiedliche Basisplatten benötigt würden. Weist die Basisplatte, beispielsweise in Breitenrichtung, beidseitig Enden eines körperumspannenden, längenverstellbaren Befestigungsgurts auf, dienen die Verlängerungsstücke durch Verbreiterung der Basisplatte zur Anpassung an den jeweiligen Körperumfang. Die erfindungsgemäß hergestellte Verbindung zwischen der Basisplatte und den angesetzten Verlängerungsstücken ist ebenfalls formstabil, und setzt daher der Zugkraft des Befestigungsgurtes die erforderliche Formstabilität entgegen, um ein unerwünschtes tiefes Einschneiden des Befestigungsgurts in das Gewebe des Patienten zu verhindern.

Es ist zur Ausbildung mehrerer verschiedener Größen der resultierenden Basisplatte zweckmäßig, wenn das Verlängerungsstück an seiner freien Kante ebenfalls mit einer Befestigungseinrichtung zur formschlüssigen Verbindung mit einem weiteren formstabilen Verlängerungsstück zur Vergrößerung der Basisplatte versehen ist. Auf diese Weise lassen sich die erwähnten mehrfachen stufenförmigen Vergrößerungen realisieren.

In einer zweckmäßigen Ausführungsform der Erfindung weist die Befestigungseinrichtung einen Ansatz mit einer stufenförmigen Ausnehmung auf und untergreift das angesetzte Verlängerungsstück die stufenförmige Ausnehmung mit einer entsprechenden stufenförmigen Ausnehmung zur Ausbildung einer teilweisen flächigen Anlage zwischen Ansatz und Verlängerungsstück, wobei in Ansatzrichtung ein Anschlag des Verlängerungsstücks an einem Anschlag des Ansatzes anliegt. Auf diese Weise wird eine sehr formstabile Verbindung ermöglicht, die durch die flächige Anlage des Verlängerungsstücks an dem Ansatz der Basisplatte verbindungssteif ist. Der Steifheit der Verbindung steht dabei nicht entgegen, dass die Verbindung zwischen Basisplatte und Verlängerungsstück und/oder zwischen Verlängerungsstücken zur Anpassung an die Körperform des Orthesenträgers um einen begrenzten Winkel kippbar ausgebildet sein kann oder dass die Ansatzstücke eine gewisse Elastizität aufweisen, sodass sie beispielsweise durch die Zugkraft eines Befestigungsgurts an dem Körper herangezogen werden können, der Verformung jedoch einen ausreichend großen Widerstand entgegensetzen, um nicht in das Gewebe des Orthesenträgers zu stark einzudrücken.

Vorzugsweise greift im montierten Zustand des Verlängerungsstücks ein senkrecht im Bereich der flächigen Anlage angeordneter Verriegelungsstift in eine entsprechende Ausnehmung zur Ausbildung der in Breitenrichtung formschlüssigen Verbindung ein. Dabei ist es zweckmäßig, wenn der senkrecht zur flächigen Anlage angeordnete Verriegelungsstift in die Ausnehmung durch eine Kippbewegung des Verlängerungsstücks relativ zum Ansatz einführbar ist, wobei der Verriegelungsstift in die Ausnehmung einrastbar gestaltet sein kann. Auf diese Weise gelingt eine stabile Verbindung, die durch ein einfaches Ansetzen und Einrasten des Verlängerungsstücks an den entsprechenden Ansatz der Basisplatte oder eines vorhergehenden Verlängerungsstücks realisiserbar ist.

Für die Ausbildung der Orthese trägt die Basisplatte vorzugsweise in Höhenrichtung wenigstens eine angesetzte Kalotte. Bevorzugt ist die Verwendung der erfindungsgemäßen Orthese als Hyperextensionsorthese mit einer Symphysenpelotte und einer Sternalpelotte. Bevorzugt sind dabei sowohl die Sternalpelotte als auch die Symphysenpelotte höhenverstellbar an der Basisplatte angebracht. Hierzu ist vorzugsweise eine Schiene aus zwei Rohren gebildet, die mit einem U-förmigen Ende im Material der Pelotte befestigt ist. Die Höhenverstellung erfolgt durch Verschieben der Rohre mit der Pelotte und durch entsprechende geeignete Befestigungsmittel der Basisplatte.

Als durch Verlängerungsstücke vergrößerbare Basisplatte kommen zahlreiche flächig am Körper des Orthesenträgers anliegende Stützplatten in Betracht, insbesondere auch die Rückenpelotte der Hyperextensionsorthese. Diese kann in einem bevorzugten Ausführungsbeispiel ebenfalls in der Breite durch Verlängerungsstücke vergrößerbar sein. Sie kann aber auch in unterschiedlichen Höhen ausgebildet werden, in dem Verlängerungsstücke, die die Breite der Rückenpelotte aufweisen angesetzt werden, um die Länge der Rückenpelotte in Höhenrichtung zu vergrößern und so eine unterschiedlich lange Unterstützung der Wirbelsäule zu realisieren.

Die erfindungsgemäße Orthese weist vorzugsweise eine Basisplatte auf, die vollständig als ein gespritztes Kunststoffteil ausgebildet ist. Entsprechend sind auch die Verlängerungsstücke gespritzte massive Kunststoffteile, vorzugsweise ohne Metallverstärkungen.

Die Erfindung soll im Folgenden anhand eines in der Zeichnung dargestellten Ausführungsbeispiels näher erläutert werden. Es zeigen:
- Figur 1: eine schematische Darstellung eines ersten Ausführungsbeispiels einer erfindungsgemäßen, als Hyperextensionsorthese ausgebildeten Orthese;
- Figur 2: eine perspektivische Teildarstellung einer Basisplatte und eines nicht montierten Verlängerungsstücks;
- Figur 3: die Darstellung gemäß Figur 2 mit dem zur Montage relativ zum Basisteil gekippten Verlängerungsstück;
- Figur 4: eine perspektivische Ansicht der proximalen Flächen der Teile gemäß Figur 3;
- Figur 5: die Teile gemäß Figur 3 während des Montagevorganges ;
- Figur 6: die Teile gemäß Figur 5 im montierten Zustand;
- Figur 7: eine schematische Darstellung eines zweiten Ausführungsbeispiels einer erfindungsgemäß als Hyperextensionsorthese ausgebildeten Orthese;
- Figur 8: eine schematische Darstellung eines dritten Ausführungsbeispiels einer erfindungsgemäß als Hyperextensionsorthese ausgebildeten Orthese.

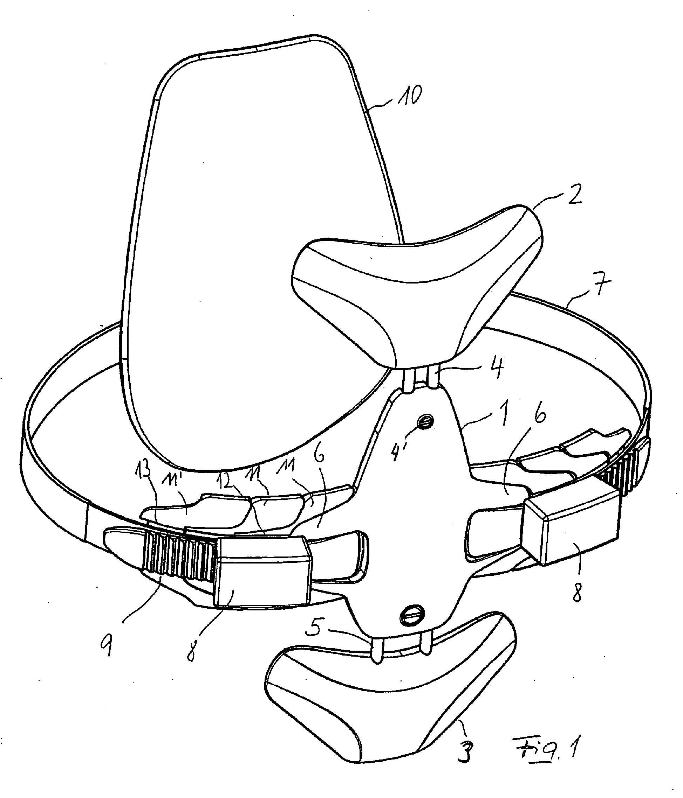

Die perspektivische Darstellung der Figur 1 einer Hyperextensionsorthese lässt eine ventrale Basisplatte 1 erkennen, an die sich in Höhenrichtung nach oben eine Sternalpelotte 2 und nach unten eine Symphysenpelotte 3 anschließen. Die Höhenposition der Sternalpelotte 2 ist einstellbar, indem die Sternalpelotte 2 auf einer durch zwei Rohre 4 gebildeten Schienenanordnung befestigt und relativ zur Basisplatte 1 verschiebbar ist. Die Basisplatte 1 weist eine Feststellschraube 4' zur Fixierung der mit der Sternalpelotte 2 verbundenen Rohre 4 in einer gewünschten Höhenstellung auf. In entsprechender Weise ist die Symphysenpelotte 3 ebenfalls höhenverstellbar an einer Schienenanordnung befestigt, die durch zwei parallele, mit einer Feststellschraube 5' fixierbare Rohre 5 gebildet ist. Die jeweils beiden parallel zueinander verlaufenden Rohre 4, 5 ergeben sich als Schenkel eines U-förmig gebogenen Rohres, wobei das U-förmige Ende der Anordnung in der betreffenden Pelotte 2, 3 befestigt ist.

Die Basisplatte 1 weist in Breitenrichtung zwei voneinander weg zeigende Ansätze 6 auf. An diesen Ansätzen 6 sind Zahnriemen 9 befestigt. Ein zur Umschlingung des Körpers eines Patienten im Taillenbereich vorgesehener Befestigungsgurt 7 weist an seinem Ende einen fest montierten Verschluss 8 auf. Der Verschluss 8 kann je nach Bedarf des Patienten auf unterschiedliche Positionen auf dem Zahnriemen 9 geschoben werden, um damit unterschiedliche Umfangsspannungen aufzubauen.

Zu der Orthese gehört eine Rückenpelotte 10, durch den der Befestigungsgurt 7 gegen den Rücken des Patienten im Brust- und Lendenwirbelbereich andrückbar ist.

Zur Anpassung der Orthese an unterschiedliche Taillenumfänge ist auf den Ansatz 6 der Basisplatte 1 ein Verlängerungsstück 11 aufgesetzt, das mit einem dem Ansatz 6 entsprechenden Ansatz 12 ausgebildet ist, sodass auf das Verlängerungsstück 11 ein weiteres Verlängerungsstück 11 usw. aufsetzbar ist. In dem in Figur 1 dargestellten Ausführungsbeispiel sind auf beiden Seiten der Basisplatte jeweils drei Verlängerungstücke 11 aufgesetzt, die - in unten näher erläuterter Weise - formschlüssig miteinander verbunden sind und eine formstabile Verbreiterung der Basisplatte 1 bewirken. Das letzte Verlängerungsstücks 11 ist als ein Abschlussstück 11' ausgebildet, das auf der distalen Oberfläche mit einer nutförmigen Führung 13 für die Führung des Befestigungsgurtes 7 ausgebildet.

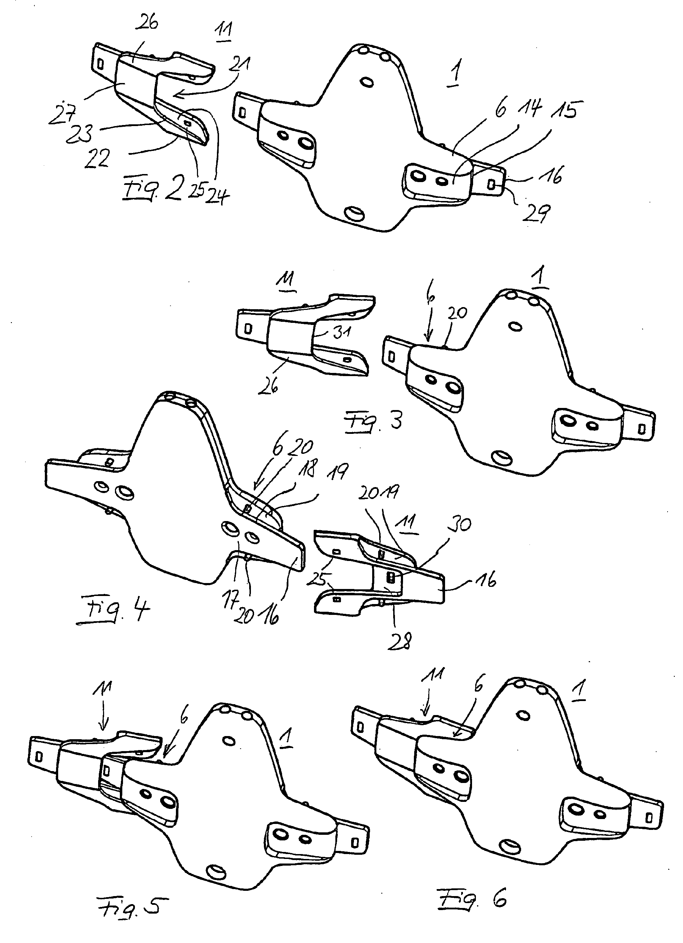

Die Figuren 2 bis 6 verdeutlichen die konstruktive Ausbildung der Basisplatte 1 und der (gleichen) Verlängerungsstücke 11. Die Figuren 2 und 3 zeigen eine perspektivische Ansicht der distalen Seiten der Basisplatte 1 und eines Verlängerungsstücks 11. Die Basisplatte 1 weist auf den Ansätzen 6 nach außen offene nutförmige Vertiefungen 14 auf, in denen die Befestigung der Enden des Befestigungsgurtes 7 mittels eines zugehörigen Schlosses 8 erfolgt. Die Ansätze 6 verjüngen sich in ihrer Breite konisch nach außen und enden in einer Kante 15, die einen Anschlag bildet. Unterhalb, also proximal von, der Kante 15 erstreckt sich eine Zunge 16 in Breitenrichtung über die Kante 15 hinaus.

Figur 4 lässt erkennen, dass die Zunge 16 auf der proximalen Seite einen Abschnitt 17 des Ansatzes 6 fortsetzt. Der Ansatz 17 erhebt sich auf der proximalen Seite stufenförmig aus dem Material des Ansatzes 6, sodass in Höhenrichtung beidseitig des Abschnittes 17 eine stufenförmige Ausnehmung 18 besteht, durch die eine stufenförmig gegenüber der Oberfläche des Abschnitts 17 zurückversetzte Anlagefläche 19 beiderseits des Abschnittes 17 ausgebildet ist. Aus der Anlagefläche 19 erhebt sich jeweils ein Verriegelungsstift 20.

Das Verlängerungsstück 11 weist ein gabelförmig ausgebildetes Ende auf, dessen Ausnehmung 21 mit einer Kontur ausgebildet ist, die der Außenkontur der Zunge 16 entspricht. Die Ausnehmung 21 wird von zwei Stegen 22 begrenzt, die auf ihrer distalen Oberfläche eine stufenförmige Übergangslinie 23 aufweisen, deren Verlauf dem Verlauf des stufenförmigen Übergangs 18 des Ansatzes 6 entspricht, also komplementär ist. Durch den stufenförmigen Übergang 23 weist jeder Steg 22 eine stufenförmig zurückversetzte Anlagefläche 24 auf, die zur Anlage an der entsprechenden Anlagefläche 19 ausgebildet ist. Die Anlagefläche 24 ist mit einer Durchgangsöffnung 25 zur Aufnahme des Verriegelungsstifts 20 versehen. Die Schenkel 22 sind jenseits der Ausnehmung 21 durch einen Grundkörper 26 miteinander verbunden, der an seiner distalen Oberseite eine nutförmige Ausnehmung 27 in Breitenrichtung aufweist und die als Führung für den Befestigungsgurt 7 dient.

Wie Figur 4 verdeutlicht, weist der Grundkörper auf seiner proximalen Seite eine mittige stufenförmige Ausnehmung 28 auf, die zur Aufnahme der über die Anschlagkante 15 hinaus stehende Zunge 16 des Ansatzes 6 dient. Die Zunge 16 ist mit einem Rastnocken 29 versehen, der in eine entsprechende Vertiefung 30 in der stufenförmigen Ausnehmung 28 im montierten Zustand eingreift.

Figur 4 verdeutlicht ferner, dass der Grundkörper 26 des Verlängerungsstücks 11 in entsprechender Weise wie der Ansatz 6 mit einer Befestigungseinrichtung aus Anlagefläche 19 und Verriegelungsstift 20 sowie mit einer Zunge 16 versehen ist, sodass der Verlängerungskörper 11 in gleicher Weise mit einem weiteren Verlängerungskörper 11 verrastbar ist.

Figur 3 verdeutlicht, dass die Verriegelung des Verlängerungskörpers 11 an dem Ansatz 6 der Basisplatte 1 dadurch erfolgt, dass das Verlängerungsstück 11 nach proximal gekippt und in dieser Stellung zum Ansatz 6 so genähert wird, dass die Anlagefläche 24 der Anlagefläche 19 gegenüberliegt Dabei liegt die Anschlagkante 15 des Ansatzes an einer entsprechenden Anschlagkante 31 des Grundkörpers 26 an. Durch Zurückkippen des Verlängerungsstücks 11 in die mit dem Ansatz 6 fluchtende Position greifen die Verriegelungsstifte 20 in die Öffnungen 25 des Verlängerungsstücks 11 ein. Gleichzeitig kommt es zu einem Eingriff des Rastnockens 29 der Zunge 16 in die Vertiefung 30 der stufenförmigen Ausnehmung 28 des Verlängerungsstücks 11. Beide Verriegelungen können rastend ausgeführt sein, sodass eine in Breitenrichtung formschlüssige Verbindung sowohl über die Verriegelungsstifte 20 als auch über den Rastnocken 29 hergestellt wird. Durch eine entsprechende Dimensionierung, beispielsweise vom Rastnocken 29 und Vertiefung 30, kann die Verbindung um eine senkrecht zur Breitenrichtung stehende Achse um einen begrenzten Winkel kippbar und/oder elastisch ausgeführt sein, wodurch selbsttätig eine Anpassung an den Körperumfang des Orthesenträgers hergestellt wird. In Zugrichtung des Befestigungsgurtes 7 besteht auch eine formstabile Verbindung aufgrund der ineinander anliegenden Anlageflächen 19, 24 auf beiden Seiten (in Höhenrichtung) des Ansatzes 6.

Figur 5 verdeutlicht die Annäherung des Verlängerungsstücks 11 an die Montageposition und Figur 6 das nach dem Zurückkippen mit dem Ansatz 6 der Basisplatte 1 fluchtend in formschlüssiger und formstabiler Weise angesetzte Verlängerungsstück 11.

Figur 6 lässt auch erkennen, dass an das Verlängerungsstück 11 ein weiteres gleich ausgebildetes Verlängerungsstück in gleicher Weise ansetzbar ist, sodass die in Figur 1 dargestellte Konfiguration mit einer durch drei Verlängerungsstücke 11 verbreitertes Basisteil 1 ohne weiteres herstellbar ist.

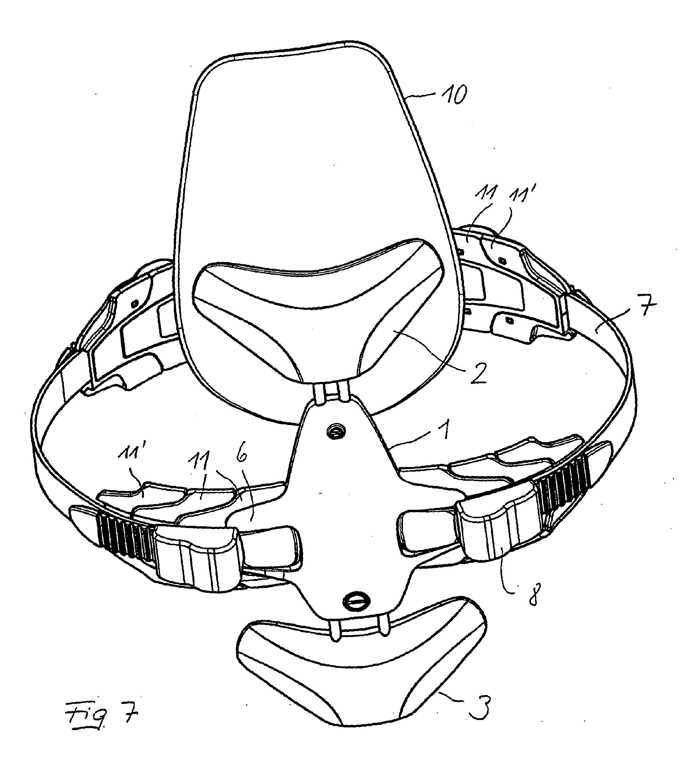

Figur 7 zeigt ein zweites Ausführungsbeispiel einer erfindungsgemäßen Orthese, die als Hyperextensionsorthese ausgebildet ist. Sie unterscheidet sich von dem ersten Ausführungsbeispiel, das in Figur 1 dargestellt ist, dadurch, dass auch die Rückenpelotte 10 eine Befestigungseinrichtung zur Befestigung von Ansatzstücken 11 aufweist, sodass auch die Breite der Rückenpelotte 10 in der gleichen Weise einstellbar ist, wie die Breite der ventralen Basisplatte 1.

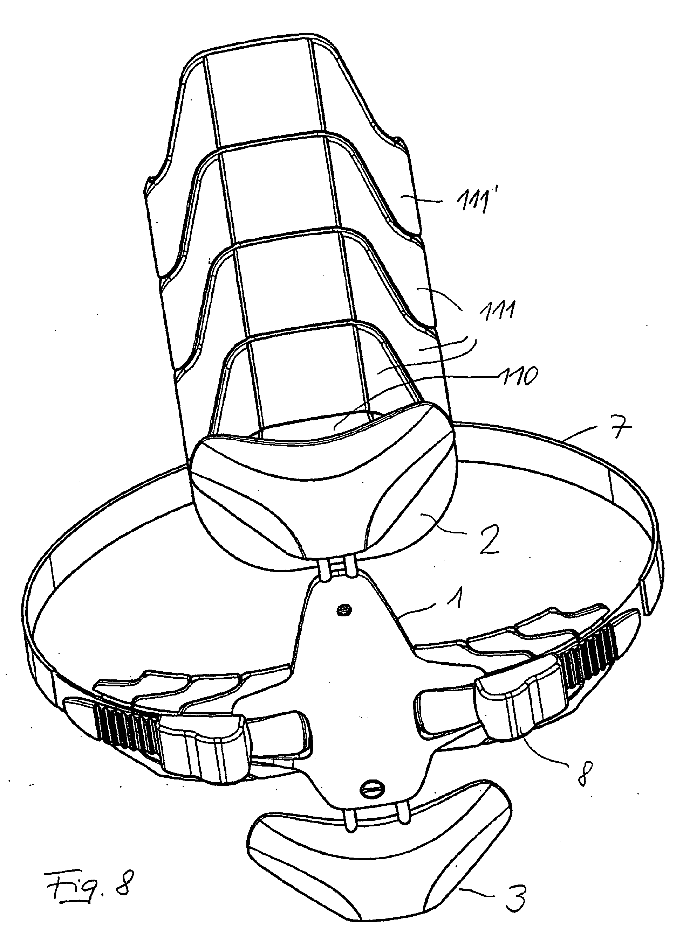

Das in Figur 8 dargestellte dritte Ausführungsbeispiel enthält gegenüber dem ersten Ausführungsbeispiel der Figur 1 die Modifikation, dass die Rückenpelotte 110 als in Höhenrichtung vergrößerbare Basisplatte ausgebildet ist. Hierzu sind an die Rückenpelotte 110 Verlängerungsstücke 111 ansetzbar, die sich über die gesamte Breite der Rückenpelotte 110 erstrecken. Auf diese Weise ist einstellbar, über welche Länge eine Abstützung der Wirbelsäule durch die Rückenpelotte 110 erfolgt. Bei dieser Ausführungsform ist es von besonderer Bedeutung, dass die Verlängerungsstücke 111 relativ zum Basisteil der Rückenpelotte 110 und relativ zueinander um einen begrenzten Winkel kippbar ausgebildet sind, sodass die Anpassung an die Kontur der Wirbelsäule erfolgen kann und dennoch die benötigte Abstützung gewährleistet ist. Ähnlich wie bei der Seitenverbreiterung wird die mittels wenigstens eines Verlängerungsstücks 111 bewirkte Verlängerung durch ein Endstück 111' abgeschlossen.

Selbstverständlich ist es auch denkbar, die breitenverstellbare Rückenpelotte 10, wie sie in Figur 7 dargestellt ist, mit der in Höhenrichtung vergrößerbaren Rückenpelotte 110 zu kombinieren, sodass die Rückenpelotte durch Verlängerungsstücke 11, 111 sowohl in Breitenrichtung als auch in Höhenrichtung vergrößerbar ist.

## Patentansprüche

1. Orthese mit einer zur Anlage am Körper des Orthesenträgers ausgebildeten Basisplatte (1), **dadurch gekennzeichnet, dass** die Basisplatte (1) an wenigstens einer Seite einen Ansatz (6) mit einer Befestigungseinrichtung (19, 20) aufweist, mit der an den Ansatz (6) ein die Basisplatte (1) vergrößerndes Verlängerungsstück (11, 11') fluchtend mit dem Ansatz (6) der Basisplatte (1) zur steifen Verbindung der Basisplatte (6) mit dem Verlängerungsstück (11, 11') angesetzt ist.

2. Orthese nach Anspruch 1, **dadurch gekennzeichnet, dass** an der Basisplatte (1) beidseitig Enden eines körperumspannenden, längenverstellbaren Befestigungsgurts (7) angebracht sind und dass wenigstens ein Verlängerungsstück (11) zur Vergrößerung der Basisplatte (1) dient, sodass die vergrößerte Basisplatte (1) der Zugkraft des Befestigungsgurts (7) einen Verformungswiderstand entgegensetzt.

3. Orthese nach Anspruch 1 oder 2, **dadurch gekennzeichnet, dass** das Verlängerungsstück (11) an seiner freien Kante ebenfalls mit einer Befestigungseinrichtung (19, 20) zur formschlüssigen Verbindung mit einem weiteren formstabilen Verlängerungsstück (11, 11') zur Vergrößerung der Basisplatte (1) versehen ist.

4. Orthese nach Anspruch 2 oder 3, **dadurch gekennzeichnet, dass** die Verlängerungsstücke (11, 11') auf ihrer distalen Oberfläche eine Führung (26) für den Befestigungsgurt (7) aufweisen.

5. Orthese nach einem der Ansprüche 1 bis 4, **dadurch gekennzeichnet, dass** die Befestigungseinrichtung (19, 20) einen Ansatz (6) mit einer stufenförmigen Ausnehmung (19) aufweist und dass das angesetzte Verlängerungsstück (11, 11') die stufenförmige Ausnehmung (19) mit einer entsprechenden stufenförmigen Ausnehmung (24) zur Ausbildung einer teilweise flächigen Anlage zwischen Ansatz (6) und Verlängerungsstück (11, 11') untergreift, wobei ein Anschlag (31) des Verlängerungsstücks (11, 11') an einem Anschlag (15) des Ansatzes (6) anliegt.

6. Orthese nach Anspruch 5, **dadurch gekennzeichnet, dass** im montierten Zustand des Verlängerungsstücks (11, 11') ein senkrecht im Bereich der flächigen Anlage angeordneter Verriegelungsstift (20) in eine entsprechende Ausnehmung (25) zur Ausbildung der formschlüssigen Verbindung eingreift.

7. Orthese nach Anspruch 6, **dadurch gekennzeichnet, dass** der Verriegelungsstift (20) in die Ausnehmung (25) durch eine Kippbewegung des Verlängerungsstücks (11, 11') relativ zum Ansatz (6) einführbar ist.

8. Orthese nach Anspruch 6 oder 7, **dadurch gekennzeichnet, dass** der Verriegelungsstift (20) in die Ausnehmung (25) einrastbar gestaltet ist.

9. Orthese nach einem der Ansprüche 2 bis 8, **dadurch gekennzeichnet, dass** die Basisplatte (1) in Höhenrichtung wenigstens eine angesetzte Pelotte (2, 3) trägt.

10. Orthese nach Anspruch 9, **dadurch gekennzeichnet, dass** sie durch eine an der Basisplatte (1) in Höhenrichtung nach unten verstellbar angebrachte Symphysenpelotte (3) und eine in Höhenrichtung nach oben verstellbar angebrachte Sternalpelotte (2) in Verbindung mit einer am Befestigungsgurt (7) angebrachten Rückenpelotte (10) zu einer Hyperextensionsorthese ausgebildet ist.

11. Orthese nach Anspruch 9 oder 10, **dadurch gekennzeichnet, dass** die wenigstens eine in Höhenrichtung verstellbare Pelotte (2, 3) an einer mit einem U-förmigen Ende im Material der Basisplatte (1) befestigten Schiene aus zwei Rohren (4, 5) höhenverstellbar befestigt ist.

12. Orthese nach einem der Ansprüche 1 bis 11, **dadurch gekennzeichnet, dass** die Basisplatte (1) und die Verlängerungsstücke (11, 11') als gespritzte Kunststoffteile ausgebildet sind.

13. Orthese nach einem der Ansprüche 1 bis 12, **dadurch gekennzeichnet, dass** die Verbindung zwischen Basisplatte (1) und Verlängerungsstück (11) und/oder zwischen Verlängerungsstücken (11, 11') zur Anpassung an die Körperform des Orthesenträgers um einen begrenzten Winkel kippbar ausgebildet ist.

14. Orthese nach einem der Ansprüche 1 bis 13, **dadurch gekennzeichnet, dass** die Basisplatte (1) und/oder das wenigstens eine Verlängerungsstück (11) mit einer eine gewisse Anpassung an die Körperkontur ermöglichenden Elastizität versehen sind, die einer Zugkraft dennoch einen dem Körper schützenden Verformungswiderstand entgegensetzt.

15. Orthese nach einem der Ansprüche 1 bis 14, **dadurch gekennzeichnet, dass** eine Rückenpelotte (10) als eine vergrößerbare Basisplatte ausgebildet ist.

16. Orthese nach einem der Ansprüche 1 bis 15, **dadurch gekennzeichnet, dass** die Rückenpelotte (10) in Breitenrichtung durch die Ansatzstücke (11, 11') vergrößerbar ist.

17. Orthese nach einem der Ansprüche 1 bis 16, **dadurch gekennzeichnet, dass** die Rückenpelotte (10) in Höhenrichtung durch die Ansatzstücke (11, 11') vergrößerbar ist.

18. Orthese nach einem der Ansprüche 2 bis 17, **dadurch gekennzeichnet, dass** die vergrößerbare Rückenpelotte (10) über den Befestigungsgurt mit der vergrößerbaren ventralen Basisplatte (1) verbunden ist.

## Claims

1. Orthosis with a base plate (1) designed to fit the body of the orthosis wearer, **characterised in that** the base plate (1) has on at least one side an attachment (6) with a fastening mechanism (19, 20) by which an extension piece (11, 11') that increases the size of the base plate (1) is attached to said attachment (6) of the base plate (1), flush therewith, for rigid connection of the base plate (1) to the extension piece (11, 11').

2. Orthosis according to Claim 1, **characterised in that** ends of a length-adjustable fastening strap (7) encircling the body are arranged on the base plate (1) on both sides, and **in that** at least one extension piece (11) serves to enlarge the base plate (1), such that the enlarged base plate (1) sets deformation resistance against the tractive force of the fastening strap (7).

3. Orthosis according to Claim 1 or 2, **characterised in that** the extension piece (11) is likewise provided at its free edge with a fastening mechanism (19, 20) for positive connection to another dimensionally stable extension piece (11, 11') to enlarge the base plate (1).

4. Orthosis according to Claim 2 or 3, **characterised in that** the extension pieces (11, 11') have a guide (26) for the fastening strap (7) on their distal surface.

5. Orthosis according to any one of Claims 1 to 4, **characterised in that** the fastening mechanism (19, 20) has an attachment (6) with a stepped recess (19) and **in that** the set extension piece (11, 11') grips under the stepped recess (19) with a corresponding stepped recess (24) to form a partially flat contact between attachment (6) and extension piece (11, 11'), whereby a stop (31) of the extension piece (11, 11') rests on a stop (15) of the attachment (6).

6. Orthosis according to Claim 5, **characterised in that** when the extension piece (11, 11') is in the assembled state a locking pin (20) arranged perpendicularly in the region of the flat contact engages in a corresponding recess (25) to form the positive connection.

7. Orthosis according to Claim 6, **characterised in that** the locking pin (20) can be guided into the recess (25) by a tilting motion of the extension piece (11, 11') relative to the attachment (6).

8. Orthosis according to Claim 6 or 7, **characterised in that** the locking pin (20) is configured to snap into the recess (25).

9. Orthosis according to any one of Claims 2 to 8, **characterised in that** the base plate (1) bears at least one board (2, 3) set in the direction of height.

10. Orthosis according to Claim 9, **characterised in that** it is designed as a hyperextension orthosis by a symphitic board (3) arranged to shift downwards on the base plate (1) in the direction of height and a sternal board (2) arranged to shift upwards in the direction of height in connection with a back board (10) arranged on the fastening strap (7).

11. Orthosis according to Claim 9 or 10, **characterised in that** the at least one board (2, 3) adjustable in the direction of height is attached height-adjustably on a rail made of two tubes (4, 5) connected to a U-shaped end in the material of the base plate (1).

12. Orthosis according to any one of Claims 1 to 11, **characterised in that** the base plate (1) and the extension pieces (11, 11') are designed as injection-moulded plastic parts.

13. Orthosis according to any one of Claims 1 to 12, **characterised in that** the connection between base plate (1) and extension piece (11) and/or between extension pieces (11, 11') is designed to tilt about a limited angle to fit the body shape of the orthosis wearer.

14. Orthosis according to any one of Claims 1 to 13, **characterised in that** the base plate (1) and/or the at least one extension piece (11) are provided with elasticity enabling a certain fit on the body contour, which sets a deformation resistance protecting the body against a tractive force.

15. Orthosis according to any one of Claims 1 to 14, **characterised in that** a back board (10) is designed as an enlargeable base plate.

16. Orthosis according to any one of Claims 1 to 15, **characterised in that** the back board (10) can be enlarged in the direction of width by the attachment pieces (11, 11').

17. Orthosis according to any one of Claims 1 to 16, **characterised in that** the back board (10) can be enlarged in the direction of height by the attachment pieces (11, 11').

18. Orthosis according to any one of Claims 2 to 17, **characterised in that** the enlargeable back board (10) is connected via the fastening strap to the enlargeable ventral base plate (1).

## Revendications

1. Orthèse comprenant une plaque de base (1) formée pour s'appuyer sur le corps du porteur d'orthèse, **caractérisée en ce que** la plaque de base (1) présente au moins sur un côté un raccord (6) ayant un dispositif de fixation (19, 20), avec lequel est jointe au raccord (6) une rallonge (11, 11') agrandissant la plaque de base (1), alignée avec le raccord (6) de la plaque de base (1), pour la liaison rigide de la plaque de base (1) avec la rallonge (11, 11').

2. Orthèse selon la revendication 1, **caractérisée en ce que** les extrémités d'une ceinture de fixation (7) réglable en longueur, entourant le corps, sont rapportées des deux côtés sur la plaque de base (1), et **en ce qu'**au moins une rallonge (11) sert à agrandir la plaque de base (1) de façon que la plaque de base (1) agrandie oppose une résistance de déformation à la force de traction de la ceinture de fixation (7).

3. Orthèse selon la revendication 1 ou 2, **caractérisée en ce que** la rallonge (11) est également munie à son bord libre d'un dispositif de fixation (19, 20) pour la liaison par formes conjuguées avec une autre rallonge (11, 11') de forme stable, pour agrandir la plaque de base (1).

4. Orthèse selon la revendication 2 ou 3, **caractérisée en ce que** les rallonges (11, 11') présentent à leur surface distale un guidage (26) pour la ceinture de fixation (7).

5. Orthèse selon l'une quelconque des revendication 1 à 4, **caractérisée en ce que** le dispositif de fixation (19, 20) présente un raccord (6) avec un évidement en forme de gradin (19) et **en ce que**, par un évidemment en forme de gradin (24) correspondant, la rallonge (11, 11') rapportée entre en prise par en-dessous avec évidement en forme de gradin (19) du raccord, pour former un appui partiellement plan entre raccord (6) et rallonge (11, 11'), une butée (31) de la rallonge (11, 11') s'appuyant contre une butée (15) du raccord (6).

6. Orthèse selon la revendication 5, **caractérisée en ce qu'**à l'état monté de la rallonge (11, 11'), une cheville de verrouillage (20) agencée perpendiculaire dans la région de l'appui plan, entre en prise dans un évidement (25) correspondant pour former la liaison par formes conjuguées.

7. Orthèse selon la revendication 6, **caractérisée en ce que** la cheville de verrouillage (20) peut être introduite dans l'évidement (25) par un mouvement de basculement de la rallonge (11, 11') relativement au raccord (6).

8. Orthèse selon la revendication 6 ou 7, **caractérisée en ce que** la cheville de verrouillage (20) est formée encliquetable dans l'évidement (25).

9. Orthèse selon l'une quelconque des revendications 2 à 8, **caractérisée en ce que** la plaque de base (1) porte dans le sens de la hauteur au moins un coussin (2, 3) rapporté.

10. Orthèse selon la revendication 9, **caractérisée en ce qu'**elle est réalisée en une orthèse d'hyperextension, par un coussin de symphyse (3) rapporté réglable en hauteur vers le bas sur la plaque de base (1), et un coussin sternal (2) rapporté réglable, en hauteur vers le haut, en liaison avec un coussin dorsal (10) rapporté sur la ceinture de fixation (7).

11. Orthèse selon la revendication 9 ou 10, **caractérisée en ce que** l'au moins un coussin (2, 3) réglable en hauteur est fixé réglable en hauteur à un rail, consistant en deux tubes (4, 5), fixé dans le matériau de la plaque de base (1) avec une extrémité en forme de U.

12. Orthèse selon l'une quelconque des revendications 1 à 11, **caractérisée en ce que** la plaque de base (1) et les rallonges (11, 11') sont formées par des pièces en matière synthétique injectées.

13. Orthèse selon l'une quelconque des revendications 1 à 12, **caractérisée en ce que** la liaison entre plaque de base (1) et rallonge (11) et/ou entre rallonges (11, 11') est formée basculante d'un angle limité, pour l'ajustement à la forme de corps du porteur d'orthèse.

14. Orthèse selon l'une quelconque des revendications 1 à 13, **caractérisée en ce que** la plaque de base (1) et/ou l'au moins une rallonge (11) sont munies d'une élasticité qui permet un certain ajustement au contour du corps, mais qui oppose à une force de traction, une résistance à la déformation protégeant le corps.

15. Orthèse selon l'une quelconque des revendications 1 à 14, **caractérisée en ce qu'**un coussin dorsal (10) est formé par une plaque de base agrandissable.

16. Orthèse selon l'une quelconque des revendications là 15, **caractérisée en ce que** le coussin dorsal (10) est agrandissable en largeur par les pièces de raccord (11, 11').

17. Orthèse selon l'une quelconque des revendications là 16, **caractérisée en ce que** le coussin dorsal (10) est agrandissable en hauteur par les pièces de raccord (11, 11').

18. Orthèse selon l'une quelconque des revendications 2 à 17, **caractérisée en ce que** le coussin dorsal (10) agrandissable est relié à la plaque de base (1) ventrale agrandissable par l'intermédiaire de la ceinture de fixation.
